# EUROPEAN PATENT APPLICATION

(11) **EP 4 597 104 A1**
(43) Date of publication of application: **06.08.2025**
(21) Application number: 23872620.2
(22) Date of filing: 29.09.2023
(51) Int. Cl.: G01N 33/50, A61K 45/00, A61P 35/00, A61P 43/00, C07K 14/47, C12N 5/09, C12N 9/12, C12Q 1/48, G01N 33/15, G01N 33/573

(54) **DRUG SCREENING METHOD BASED ON DRUG DISCOVERY CONCEPT OF CANCER CELL PROLIFERATION INHIBITION INDUCED BY DISRUPTION OF CHROMOSOME REGULATION DUE TO PLK1 KINASE ACTIVATION**

(30) Priority: 29.09.2022 JP 2022156506
(71) Applicant: Japanese Foundation For Cancer Research, Tokyo 135-8550 (JP)
(72) Inventor: HIROTA Toru, Tokyo 135-8550 (JP); TAKAHASHI Motoko, Tokyo 135-8550 (JP); JO Minji, Tokyo 135-8550 (JP); KATO Utako, Tokyo 135-8550 (JP); KAMAKURA Nana, Tokyo 135-8550 (JP); KAWAKITA Norika, Tokyo 135-8550 (JP)
(74) Representative: Schön, Christoph
(86) International application number: PCT/JP2023/035706
(87) International publication number: WO 2024/071401

(57) **Abstract**

[Abstract] It was found that enhancement of Plk1 activity induces lethal abnormalities in chromosome dynamics in cancer cells, causing the growth to be suppressed. Therefore, activating Plk1 and exacerbating chromosome instability can lead to lethal abnormalities in chromosome dynamics and suppress the growth of cancer cells. Provided are a method for screening a medicine and a pharmaceutical composition based on a new drug discovery concept in which activating a kinase that exhibits enhanced expression in cancer cells causes the suppression of cancer cell growth.

## Description

### Technical Field

The present invention relates to a method for screening anticancer agents that target polo-like kinase 1 (Plk1) and a method for suppressing cancer cell growth.

### Background Art

Cancer chemotherapy is said to be one of the three major options for cancer treatment, along with surgical resection and radiotherapy. Chemotherapy using anticancer agents has been used in different situations, for example, used alone, before or after surgery, or in combination with radiotherapy. In recent years, numerous molecular targeted drugs have been developed as substitutes for cytotoxic anticancer drugs. For example, antibody medicines that target receptors and ligands thereof present on cell membranes, or differentiation antigens, have been developed as molecular targeted drugs. Moreover, compounds that target signal transduction and proteasomes as molecular targets in cells and inhibit their functions have been developed as molecular targeted drugs. In particular, kinase inhibitors against various kinases have been developed as molecular targeted drugs.

Kinases that are activated during the cell division (M phase), such as Aurora kinases and Plk1 kinases are overexpressed in many cancers, and their expression correlates with the malignancy, therefore, the kinase inhibitors thereof have been expected to be anticancer drugs. However, no agents that can be clinically used have yet been developed.

The regulation of chromosome dynamics in the M phase is based on the binding regulation between kinetochores and microtubules, and the chromosome dynamics are regulated through an appropriate balance between two kinases with opposing roles: Aurora B, which destabilizes the binding between the kinetochores and microtubules, and Plk1, which stabilizes it. The M phase kinase Plk1 is known to be overexpressed in different cancer types, and among others, the overexpression in lung adenocarcinoma, bladder cancer, and clear cell renal carcinoma has been reported to be poor prognosis factors (Non Patent Literature 1). Against such a background, the development of Plk1 kinase inhibitors has developed, and the inhibitors have been expected to be promising as a new anticancer drug; however, development of agents that can be clinically used has not yet succeeded (Patent Literatures 1 to 3). For example, BI2536 exhibited efficiency in non-clinical trials, but its development was stopped at Phase II due to its poor activity. Also, the development of BI6727/Volasertib in which the inhibitory effect was enhanced progressed to Phase III for acute myeloid leukemia; however, its clinical application has not been made because serious side effects such as leukopenia appeared.

As described above, the stable segregation of chromosomes in the M phase is achieved by the action of microtubule destabilizer and stabilizer in the kinetochore, and appropriate binding is achieved by dynamic regulation of the balance between the two. For example, when the destabilizer becomes strong due to microtubule agonists, the binding efficiency between the microtubules and the kinetochores decreases, and therefore a checkpoint is activated to stop the M phase; whereas when the stabilizer is strengthened, binding errors that should be resolved remain to cause chromosome segregation errors (Non Patent Literature 2). What is important is that the stabilizer is dominant in this balance regulation in many cancer cells, and therefore, it has been pointed out that frequent occurrence of abnormalities in chromosome segregation in the M phase is the main mechanism explaining the chromosome instability (Non Patent Literatures 3 and 4).

### Citation List

### Patent Literature

Patent Literature 1:
   International Publication No. WO 2006/025567
Patent Literature 2:
   International Publication No. WO 2006/049339
Patent Literature 3:
   International Publication No. WO 2008/081914

### Non Patent Literature

Non Patent Literature 1:
   de Carcer., 2019, Genes 10, 208; doi:10.3390/genes10030208
Non Patent Literature 2:
   Liu et al., 2012, J Cell Biol. Vol. 198, pp.491-499
Non Patent Literature 3:
   Thompson et al., 2008, J Cell Biol. 180: 665-672
Non Patent Literature 4:
   Abe et al., 2016, Dev Cell, Vol. 36. pp.487-497
Non Patent Literature 5:
   Xu et al., 2013, Nat Struct Mol Biol, Vol. 20(9), pp.1047-53
Non Patent Literature 6:
   Zhu et al., 2016, Structure Vol. 24, pp.1110-1119
Non Patent Literature 7:
   Jand et al., 2002, J Biol Chem. Vol. 277, pp. 44115-44120. doi:10.1074/jbc.M202172200

### Summary of Invention

### Technical Problem

The present inventors have been studying chromosome regulations with the M phase kinase as the keystone. The "chromosome instability", in which the number of chromosomes increases or decreases each time cells divide is a cellular pathological condition into which many advanced cancer cells commonly fall. The growth of aneuploid cells in which the number of chromosomes vastly varies due to chromosome instability is considered to accelerate the heterogeneity of cells that make up cancer tissue and make cancer treatment difficult. On the other hand, the increase and/or decrease in the number of chromosomes itself has a strong suppressing effect on cell growth because of chromosome stress involved. Intervening in the molecular mechanism of the regulation of chromosome dynamics in the M phase and exacerbating the chromosome instability of cancer cells could lead to the growth suppression of the cancer cells due to excessive chromosome stress.

The present inventors have revealed that, in many cancer cells, regulatory imbalance in which the stabilization becomes dominant due to a decrease in the function of Aurora B, resulting in chromosome instability. The present invention has focused on this imbalance state and established a methodology for disrupting the regulation of binding between kinetochores and microtubules to induce lethal abnormalities in chromosome dynamics. In other words, it is an approach to further increase the level of imbalance by enhancing the Plk1 activity in cancer cells, exacerbate the chromosome instability, and induce lethal abnormalities in chromosome dynamics to suppress the growth of the cancer cells. Conventional molecular targeted drugs that target kinases are "inhibitors" that suppress the activity of the kinases. In contrast, the present invention is based on a completely new concept of aiming to suppress cancer growth by enhancing the Plk1 activity. The present invention focuses on the chromosome instability, which becomes prominent along with cancer progression, and relates to a method for screening medicines based on the molecular mechanism thereof.

### Solution to Problem

The present invention relates to the following screening method and a method for suppressing cancer cell growth.
(1) A method for screening a medicine that targets polo-like kinase 1 (Plk1), in which Plk1 activation by addition of a candidate compound is used as an indicator.
   All molecular targeted drugs against kinases that have been developed so far are inhibitors. In contrast, the present inventors have found that constitutive activation of Plk1 induces lethal abnormalities in chromosome dynamics and eventually cell death. Molecular targeted drugs that induce cell death by kinase activation represent a new drug discovery concept that has never been seen before. Specific methods are described below, and known methods for allowing a candidate compound to act on Plk1 to detect kinase activation such as FRET, kinase assay, and protein phosphorylation can be used for screening. When a Plk1 recombinant protein is used, a candidate compound can be selected with high efficiency by high-throughput screening.
(2) The screening method according to (1), in which the Plk1 activation is due to inhibition of an association between a Plk1 kinase domain and a Polo-Box domain (PBD).
   Since the Plk1 activation occurs by inhibiting its intramolecular association, molecular targeted drugs can be screened by selecting a compound that inhibits it.
(3) The screening method according to (1) or (2), in which the Plk1 activation is measured by A FRET assay, a kinase assay, or an antibody that detects phosphorylation.
   The Plk1 activation can be detected by any method as long as the inhibition of the intramolecular association can be detected, and it can be sensitively detected by a FRET assay, a kinase assay, or Plk1 phosphorylation.
(4) The screening method according to (1), in which the medicine is for treating cancer.
   To date, anticancer agents have been developed that target and inhibit kinases activated in the M phase; however, there are no medicines that have been clinically used. According to the present screening method based on the new drug discovery concept, lethal abnormalities in chromosome dynamics can be induced in cancer cells.
(5) A method for suppressing cancer cell growth, in which the growth is suppressed by activating Plk1.
   Activating Plk1 can cause the growth of cancer cells to be suppressed. Cell growth suppression by activating kinases is a completely new technique.
(6) A pharmaceutical composition for treating cancer, in which a compound that activates Plk1 is used as an active ingredient.
   As shown herein, activating Plk1 and disrupting the chromosome dynamics-regulating functions can cause lethal chromosome stress to cancer cells. As a result, cancer cell-specific growth arrest and cell death can be induced. Accordingly, the compound that activates Plk1 can be used as an active ingredient of the pharmaceutical composition for treating cancer.
(7) The pharmaceutical composition for treating cancer according to (6), in which a partner of numb (PON) peptide or a nucleic acid that encodes a PON peptide is used as an active ingredient.
   As shown in the following Examples, expressing the PON peptide in cancer cells can induce cell death. Accordingly, the PON peptide or the nucleic acid that encodes the PON peptide can be used as a therapeutic drug for cancer. As the PON peptide or the nucleic acid that encodes the same, the sequence set forth in SEQ ID NO: 1 below, a region of a PON protein containing the PON peptide, a full-length PON protein or a nucleic acid that encodes the same, specifically DNA or RNA can be used. In addition, a peptide modified to have higher Plk1-binding may be used.
(8) A method for treating cancer, comprising administering the pharmaceutical composition according to (6) or (7).
   Administering the PON peptide or the nucleic acid that encodes the PON peptide can cause cell death and cell growth suppression to be induced specifically in cancer cells, enabling treating cancer. Any administration method can be used as long as a peptide or a nucleic acid can be delivered to a tumor. Specifically, known methods including oral administration, subcutaneous administration, intravenous administration, intraperitoneal administration, and intratumoral administration can be used. Also, the dosage can vary depending on the subject to be administered, the mode of administration, the administration form, or the like, and those skilled in the art can determine a therapeutically effective amount using a known method as appropriate. In addition, the treatment method is considered to be effective against all cancers, and to be more effective against cancers in which activation and/or overexpression of Plk1 are observed. Consequently, it is considered that a higher effect can be obtained if Plk1 expression or Plk1 activation is analyzed in a cancer region prior to treatment, and cancers in which high expression is observed are treated.

### Brief Description of Drawings

[Figure 1A] Figure 1A is a diagram showing the correlation between Plk1 expression levels and clinicopathological characteristics in clinical specimens.
[Figure 1B] Figure 1B is a graph showing the correlation between Plk1 expression levels and survival rates.
[Figure 2] Figure 2 is a graph showing the effects of constitutively active Plk1 mutants on cell death.
[Figure 3] Figure 3 is a graph showing abnormalities in chromosome dynamics due to constitutively active Plk1 mutants.
[Figure 4A] Figure 4A is photographs showing abnormalities in chromosome dynamics of cells expressing Plk1 intramolecular association-suppressing mutants.
[Figure 4B] Figure 4B is a graph showing the effects of the expression of Plk1 intramolecular association-suppressing mutants.
[Figure 5] Figure 5 is a diagram schematically showing a drug discovery concept.
[Figure 6A] Figure 6A is diagrams schematically showing structures of inactive and active forms of Plk1.
[Figure 6B] Figure 6B is a graph showing the effects of PON peptide expression on cell death.
[Figure 7A] Figure 7A is diagrams showing structures of Plk1 recombinant protein for use in a FRET assay and kinase assay (left) and the purity of purified protein.
[Figure 7B] Figure 7B is a graph showing results of time-resolved fluorescence-fluorescence resonance energy transfer using Plk1 recombinant proteins and PON peptides.
[Figure 8] Figure 8 is diagrams schematically showing recombinant constructs for performing an intramolecular FRET or intermolecular FRET assay.
[Figure 9] Figure 9 is a graph showing the results of a FRET assay using wild-type and mutant Plk1 recombinant proteins.
[Figure 10A] Figure 10A is a graph showing the results of a kinase assay using Plk1 recombinant proteins.
[Figure 10B] Figure 10B is a graph showing the results of a kinase assay using wild-type and mutant Plk1 recombinant proteins.

### Description of Embodiments

The present invention will be described with reference to drawings.

### [Plk1 Expression in Clinical Specimen]

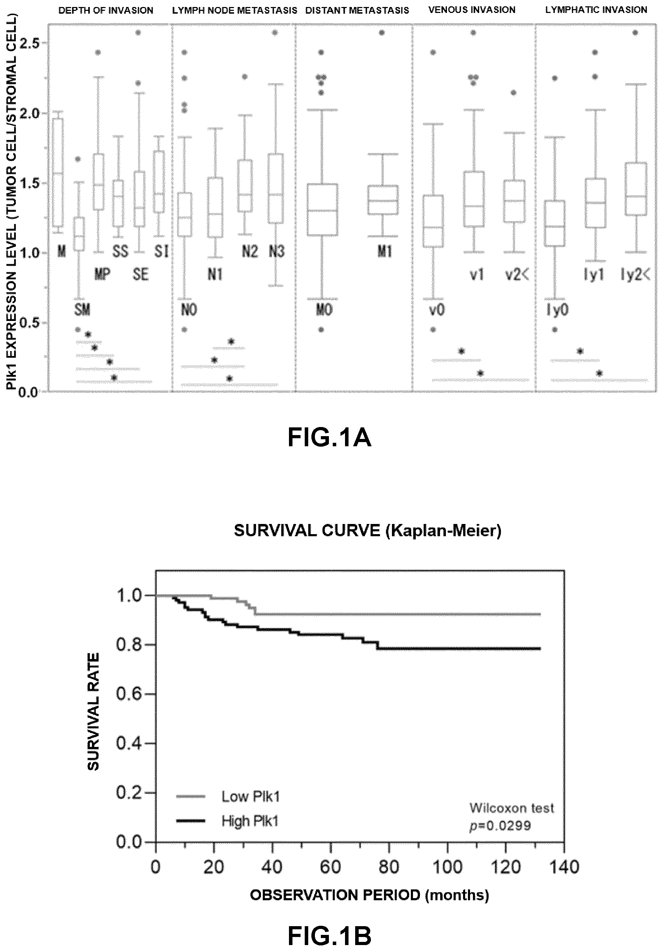

The Plk1 expression in clinical specimens was first examined. Tissue microarrays were prepared from tumor resection samples of a plurality of gastric cancer cases, and immunostained using a Plk1 antibody (F-8 monoclonal antibody, Santa Cruz Biotechnology, Inc.). Using publicly available software QuPath, the Plk1 expression levels of tumor cells and stromal cells were quantified on each tumor sample by the degree of depth of invasion, lymph node metastasis, distant metastasis, vascular invasion, and lymphatic invasion of tumor. The ratio of the expression levels of the tumor cells to the expression levels of the stromal cells was calculated, and the correlation on each item was analyzed. An increased Plk1 expression (tumor cells / stromal cells > 1) was significantly correlated with the depth of invasion reaching the muscularis propria or deeper, the lymph node metastasis of N2 or more, and the vascular invasion of the tumor (Figure 1A).

Further, the correlation between the Plk1 expression and the prognosis was analyzed (Figure 1B). Cases where the ratio of Plk1 expression level of the tumor cells to stromal cells was 1.27 or more were classified as High Plk1 (107 cases), and the others as Low Plk1 (82 cases), and then the survival rates were analyzed, revealing that the High Plk1 patient group had a poorer prognosis. Examination using clinical specimens suggested a correlation between Plk1 activity and clinical malignancy.

### [Constitutive Activation of Plk1 Leads to Lethal Abnormalities in Chromosome Dynamics]

As described above, the chromosome dynamics in the M phase are regulated through an appropriate balance between two kinases with opposing roles: Aurora B, which destabilizes the binding between kinetochores and microtubules, and Plk1, which stabilizes it. In addition, although the development of Plk1 kinase inhibitors has progressed, the clinical application has not been achieved. It is considered that lethal abnormalities in chromosome dynamics are induced when the balance between the two kinases Aurora B and Plk1 is lost, which may induce apoptosis in cancer cells. Therefore, a working hypothesis that "activating Plk1 and exacerbating the chromosome instability lead to lethal abnormalities in chromosome dynamics and suppress the growth of the cancer cells" was made and verified.

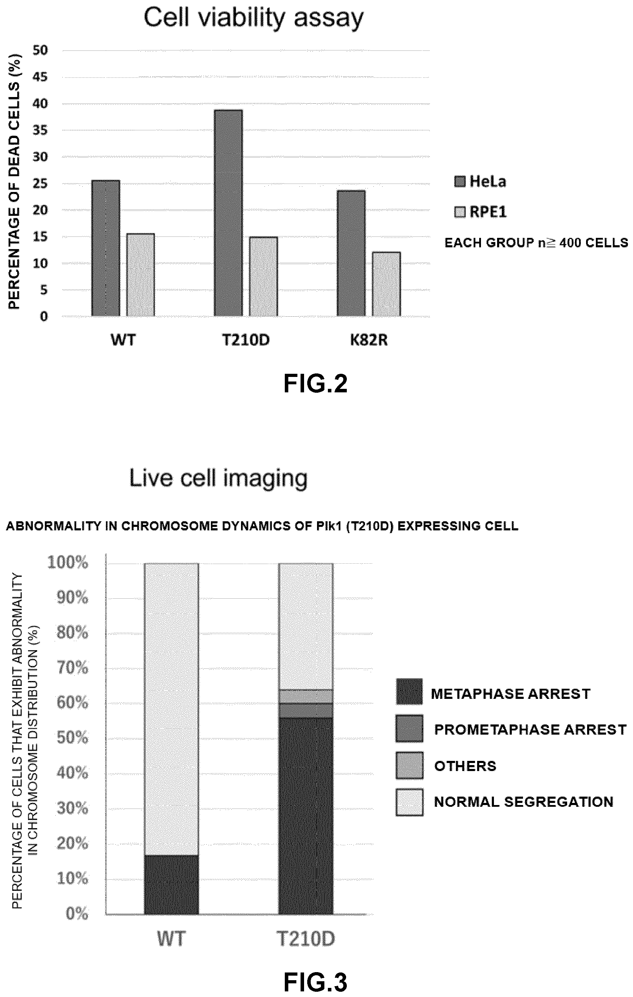

To evaluate the effect of constitutively active Plk1, wild-type, constitutively active, and kinase inactive mutants were overexpressed in HeLa cervical cancer cells and retinal pigment epithelial cells RPE 1, which are normal diploid cells, and the percentage of dead cells was determined. Wild-type Plk1, the constitutively active mutant T210D in which phosphorylation was mimicked by substituting Thr at position 210 with Asp, and the kinase inactive mutant K82R which had a mutation at an ATP binding site and had an mCherry tag added to the N-terminus, were overexpressed. Propidium iodide (PI) staining was performed 48 hours after each construct was introduced, and the number of dead cells stained with PI in the cells expressing mCherry was counted. The viability was determined in 400 or more cells for each group, and the percentage of dead cells was determined (Figure 2). The overexpression of the constitutively active mutant T210D was lethal in the HeLa cells, whereas the overexpression of the inactive mutant did not result in increased dead cells. Also, no effect of the active Plk1 was observed, in the normal diploid cells RPE1. The lethal effect of Plk1 activation was prominently observed in cancer cells, suggesting that the effect on normal cells was minimal.

Next, the inventors examined whether the cell death due to the expression of the constitutively active Plk1 mutant was caused by abnormalities in chromosome dynamics. The constitutively active Plk1 mutant T210D in which its expression was induced by the Tet expression induction system was introduced into the HeLa cells to observe chromosome dynamics by live cell imaging (Figure 3). Specifically, cell lines were prepared in which mCherry-Plk1 (WT and T210D mutants) having a synonymous mutation sequence that was not recognized by siRNA, was each introduced into HeLa TetON cells. Endogenous Plk1 was knocked down by RNAi, and at the same time, the expression of the introduced Plk1 was induced by the addition of doxycycline (Dox). DNA was stained with a DNA staining reagent SiRDNA, and live cell imaging was performed to observe chromosome dynamics. As shown in Figure 3, it was indicated that the progression of mitosis was significantly delayed by the expression of the T210D mutant and constitutive activation of Plk1, and the frequency of abnormalities in chromosome dynamics was increased. These results indicate that enhanced Plk1 kinase activity leads to an imbalance in the regulation of microtubule dynamics, resulting in lethal abnormalities in chromosome dynamics.

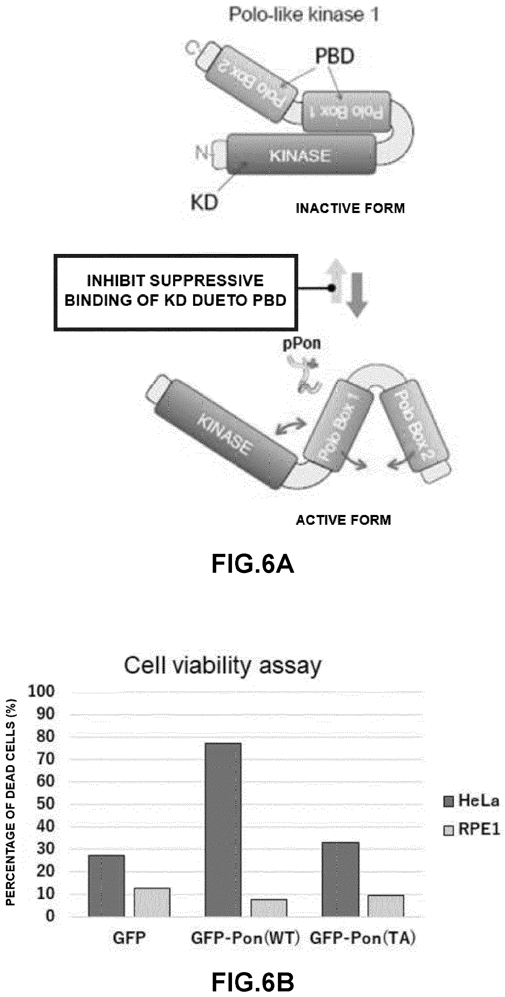

Inhibiting suppressive association between kinase domain (KD) and Polo-Box domain (PBD) causes Plk1 to be constitutively activated. (see Figure 6A). Based on structural information on the binding between the kinase domain and PBD in the Plk1 molecule, amino acids important for the interaction were substituted and mutants that inhibit suppressive association were produced. S137D and R144E were produced as constitutively active Plk1 mutants. *In vitro* measurements predict that Plk1 kinase activity increases about 10-fold or more in these mutants (Non Patent Literature 5).

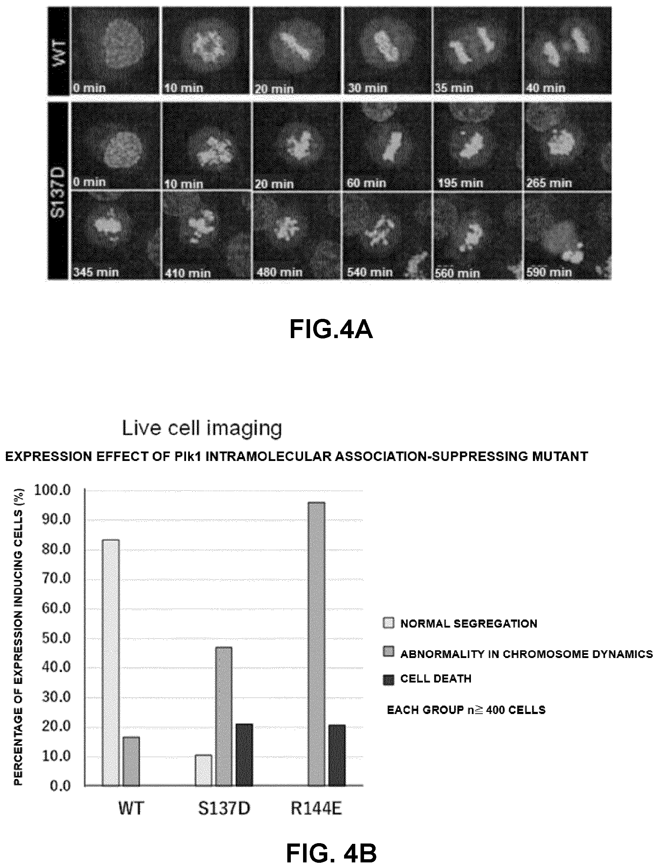

As described above, cell lines were prepared in which mCherry-Plk1 (WT, S137D mutant, and R144E mutant) having a synonymous mutation sequence that was not recognized by siRNA, was each introduced into HeLa TetON cells. Endogenous Plk1 was knocked down by RNAi, the expression of the above Plk1 was induced by the addition of doxycycline at the same time, and 5 hours later, live cell imaging was performed to observe the chromosome dynamics. Abnormalities in chromosome dynamics due to abnormal binding between kinetochores and microtubules, and the resulting disruption of cell division and cell death were reproducibly observed (Figure 4A). When intramolecular association mutants, S137D mutant and R144E mutant, which constitutively activate Plk1, are expressed, abnormalities in chromosome dynamics and cell death are induced at a high rate (Figure 4B). These results indicated that activating Plk1 induces lethal abnormalities in chromosome dynamics in cancer cells, causing cell death.

### [Examination of Potential for Drug Discovery]

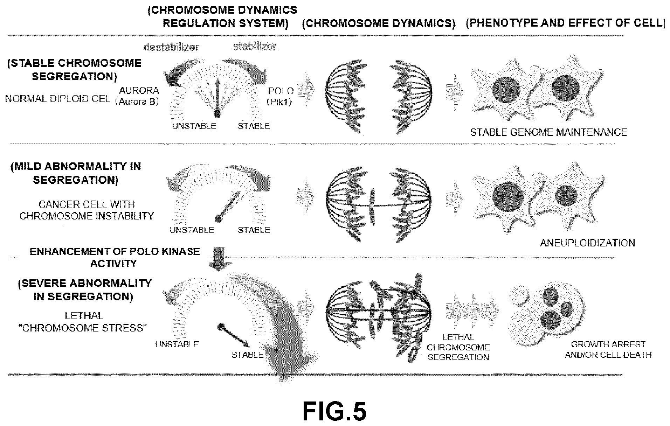

As described above, the overexpression of Plk1 is observed in cancer cells, and further, activating Plk1 expressed in the cancer cells can induce cell death due to lethal abnormalities in chromosome dynamics. Using a new drug discovery concept based on this experimental result, a therapeutic drug for cancer can be created. As shown in Figure 5, in the normal diploid cells in which the chromosome dynamics regulation system functions normally, genome maintenance is stably performed by stable chromosome segregation. On the other hand, in the cancer cells, the balance between Aurora B and Plk1, the chromosome dynamics regulation system, becomes impaired, resulting in mild abnormalities in segregation. As a result, chromosome instability occurs, leading to aneuploidization of the cancer cells. To cancer cells in which the Plk1 activation is observed, Plk1 is further activated, and hence, lethal chromosome segregation can be induced in cancer cells, causing cell arrest and cell death. Conventionally, compounds inhibiting kinases that are activated during cell division, such as Aurora kinase and Polo kinase, have been considered for use in cancer treatment and developed. The drug discovery concept presented herein is a completely new drug discovery concept, which involves inducing cell death by activating Plk1 and causing lethal chromosome stress to cancer cells. In other words, it involves converting wild-type Plk1 expressed in cancer cells into an active form and inducing cell death specifically in the cancer cells.

For that, the inventors verified whether endogenous Plk1 can be activated to induce lethal abnormalities in chromosome dynamics and cell death could be induced, based on this new drug discovery concept. A peptide consisting of 15 amino acids that binds to Plk1 and maintains an active form (portion of a protein called PON) is known (Non Patent Literature 6). Crystal structure analysis indicates that the PON peptide binds to PBD of Plk1 and has a preventive effect on suppressive association of the PBD with the kinase domain (KD), and as a result, Plk1 becomes an active form (Figure 6A). Therefore, the inventors examined whether lethal abnormalities in chromosome dynamics and cell death were induced by the PON peptide.

Plasmids that express WT (ESCFTNAAFSSTPKK: SEQ ID NO: 1) and a TA mutant (Plk1 unbound, ESCFTNAAFSSAPKK: SEQ ID NO: 2) of the PON peptide with a GFP tag added to the N-terminus were introduced into HeLa cells by a plasmid transfection method with FuGENE HD transfection Reagent (Promega Corporation) and into RPE1 cells by a nucleofection method with 4D nucleofector (Lonza Group Ltd.). Since the PON peptide binds to the PBD upon phosphorylation by Cdk1, a TA mutant of the PON peptide in which the phosphorylation site was substituted with Ala was used as a negative control.

Observation was performed using live cell imaging, and the number of cells that underwent cell death in cells with GFP expression observed was counted. In the HeLa cells, the expression of the PON peptide caused the death of 70% or more of cells, whereas the expression of the TA mutant caused the death of only about 30% of cells, which indicated that no significant difference was observed from the percentage of cell death in the control GFP expressing cells. Also, even though the PON peptide was introduced into the RPE1 cells, no increase in cell death was observed. In other words, suppression of the association of the PBD and KD of endogenous Plk1 can cause significant cell growth suppression to be induced in cancer cells (Figure 6B). From the fact that induction of cell death due to Plk1 activation is not observed in normal cells, compounds that activate Plk1 are expected to exert an effect in a cancer cell-specific manner. The above results indicate that cancer can be treated by expressing DNA or RNA that expresses PON peptides in cancer cells.

Any nucleic acid can be used to introduce into cells as long as it can express a PON peptide, or a peptide that is similar to the PON peptide and that binds to Plk1. Examples thereof include nucleic acids that express a peptide containing the PON protein (SEQ ID NO: 3, NP_525072.2), amino acids at positions 1 to 116 that contain the PON peptide and that have been confirmed to strongly bind to Plk1, and the PON peptide (at positions 52 to 66, SEQ ID NO: 1). Also, examples of peptides similar to the PON peptide include peptides in which threonine at position 63, which is a phosphorylation site important for Plk1 binding, is substituted with an amino acid that mimics phosphorylation. In addition, the nucleic acid can be administered by a known method, delivered to a cancer site, and used for treatment.

### [Construction of Screening System]

Since it was indicated that activating Plk1 can cause the growth of cancer cells to be suppressed, a drug discovery screening system was constructed. In other words, it is an assay system that reflects the folding state within Plk1 molecules, and a screening system for drug discovery sees according to the mechanism of action of "inhibiting the suppressive association within the molecules to lead to kinase activation". A fluorescence resonance energy transfer (FRET) assay system that detects the interaction of PBD that suppressively associates with the Plk1 kinase domain was constructed. The design was made as follows: a full-length recombinant protein was purified, and a donor (GST-Tb) were placed in the kinase domain and an acceptor (His-d2) in the PBD using antibodies that act as luminescent agents based on the conformational information so that the FRET reaction would efficiently occur.

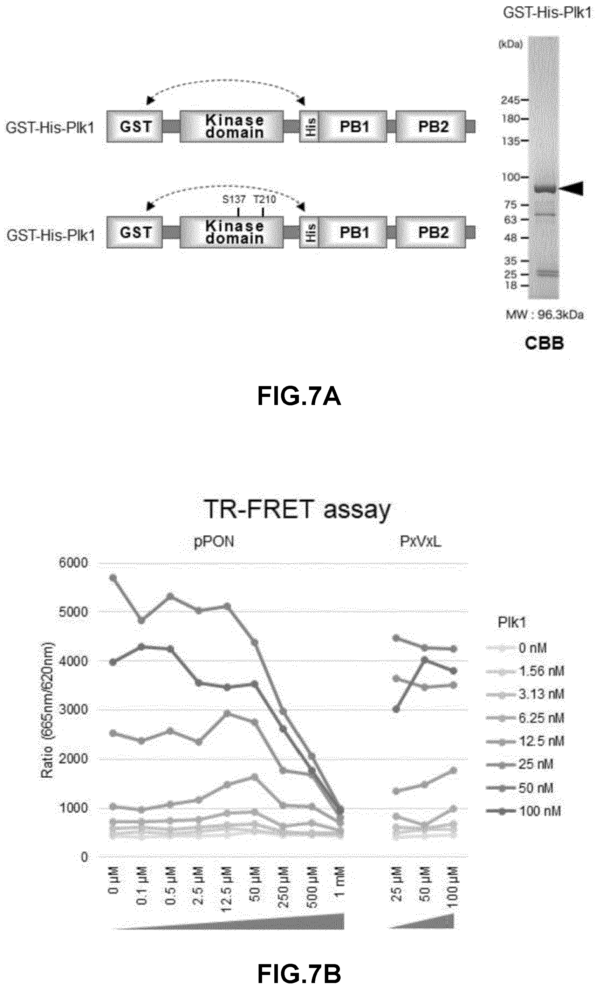

A recombinant Plk1 in which a GST tag was added into a pFastBac1 vector at the N-terminus, and a 6His-tag was inserted in the vicinity of the N-terminus of PB1 (Polo-Box 1) was constructed (left in Figure 7A, GST-His-Plk1). Since the recombinant protein prepared from E. coli required many column purification steps, and the yield, concentration and purity of the finally obtained protein were low, preparation was performed using a Baculovirus-insect cell system. As a result, it was possible to obtain a sufficient amount of recombinant proteins with high purity (right in Figure 7A).

Specifically, the GST-His-Plk1 was transformed into DH10Bac, bacmid was produced and transfected with Sf9, and the culture supernatant was collected on Day 5 as a P1 virus. The P1 virus was used to infect Sf9, and the culture supernatant was collected 72 hours later as a P2 virus. The P2 virus was used to infect Sf9, the cells were collected 60 hours later, and the recombinant protein was purified using Glutathione Sepharose 4b resin.

Intramolecular FRET analysis was performed using the recombinant GST-His-Plk1. Plk1 (0 to 100 nM) was reacted with pPON (SEQ ID NO: 1, 0 to 1000 µM) and a peptide that does not bind to PLK1 (PxVxL, negative control, 25 to 100 µM, NHTQCQLVPVVEIGISERQN, SEQ ID NO: 4) at room temperature for 1 hour. Then, GST-Tb and His-d2 (PerkinElmer, Inc., MAb Anti-GST-Tb cryptate, MAb Anti-6HIS-d2) were added thereto to react at room temperature for 3 hours, and the emission signals were measured using the time-resolved fluorescence-fluorescence resonance energy transfer method (TR-FRET). An emission signal is detected in an inactive state where the GST tag and 6His-tag, that is, the kinase domain and Polo-Box domain are located closely (see Figure 6A). The His-d2 signals are shown relative to GST-Tb signals (Figure 7B). As the amount of pPON peptide added increased, FRET signals were reproducibly observed to gradually decrease; while this effect was not observed in the control peptide that does not bind to Plk1.

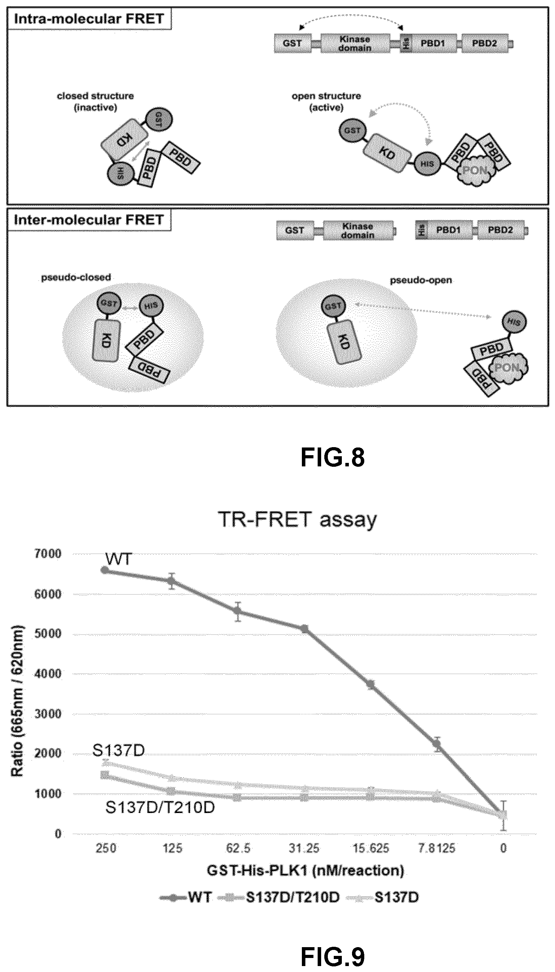

Here, intramolecular FRET assay results are shown, but similar results were also obtained in the case where two recombinant proteins, a GST-kinase domain and a His-PBD, were produced (Figure 8), and an intermolecular FRET assay was performed. In addition, an example of a construct used for FRET is shown here, but the arrangement of the donor and acceptor, and illuminant can be changed as appropriate, as long as the association between the kinase domain and PBD, and suppression of the association can be detected.

S137D/T210D and S137D mutants (see Figure 7A), which are expected to be open without binding of the kinase domain and PBD of Plk1, were each produced and used as controls, and then, the FRET signals of WT were measured. To each recombinant GST-His-Plk1 (WT, S137D/T210D mutant, and S137D mutant) (0 to 250 nM), GST-Tb and His-d2 were added, and the mixture was reacted at room temperature for 3 hours to detect emission signals of GST-Tb and His-d2. The His-d2 signals are shown relative to GST-Tb signals. In the wild-type GST-His-Plk1, signals were detected in a concentration-dependent manner, whereas no enhancement of signals was detected in the S137D/T210D mutant and S137D mutant (Figure 9).

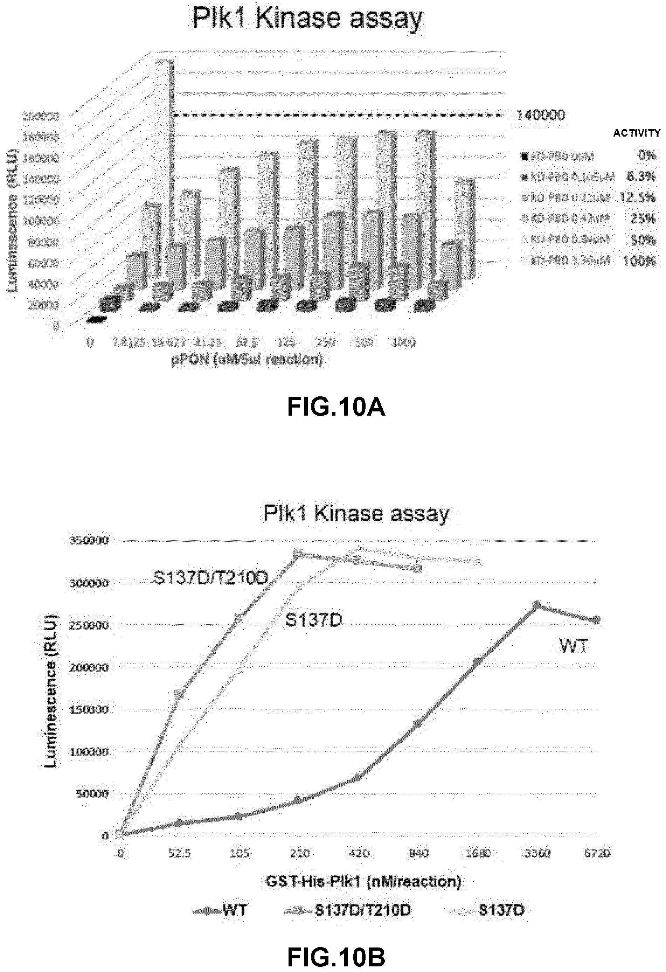

Further, the kinase activity of GST-His-Plk1 was evaluated by a kinase assay. First, it was confirmed that the kinase activity of the recombinant GST-His-Plk1, which contained a His-tag for FRET in the molecule, was equivalent to that of the one without the tag. Next, GST-His-Plk1 was used and mixed with 0.5 µg of dephosphorylated casein as a phosphorylation substrate in the presence of pPON (0 to 1000 µM), a kinase reaction was performed at room temperature for 1 hour in the presence of 5 µM of ATP, and a kinase reaction was performed between the recombinant GST-His-Plk1 (WT) (0 to 3.36 µM) and the dephosphorylated casein. Using an ADP-Glo (trademark) kinase assay kit (Promega Corporation), the kinase activity was measured by an ADP-Glo method, which quantifies in the amount of ATP consumed. As a result, it was observed that the kinase activity increased depending on the amount of pPON added (Figure 10A). Also, the results of the kinase assays using the S137D/T210D mutant and S137D mutant also indicate that high kinase activity can be obtained when the intramolecular association is inhibited (Figure 10B). Accordingly, the kinase assay can be used as a screening method that detects the association between the kinase domain and PBD of Plk1, and suppression of the association can be detected. Also, in the case of using the kinase assay for screening, a construct in which a kinase domain and PBD are separated can also be used.

Further, a screening system can also be constructed using an antibody that detects Plk1 activation. It is known that Plk1 activation causes the T-loop region to be phosphorylated (Non Patent Literature 7). Since an antibody that specifically detects the phosphorylation of threonine at position 210 has already been commercially available, for example, this can be used for screening a medicine that enhances the Plk1 activity. Also, an antibody that specifically detects the Plk1 activation may be newly produced for use. Specifically, a candidate compound can be added to a culture solution of cultured cells, or a candidate peptide can be introduced to detect Plk1 phosphorylation using an antibody. Known detection methods, such as Western blotting and immunostaining can be used.

As described above, it was confirmed that the pPon peptide enhances the Plk1 activity overall by abolishing the suppressive binding of the kinase domain by PBD. This means that an assay system was able to be constructed to detect the "regulation of the kinase activity by intramolecular structural conversion of Plk1". It was found that activating Plk1 induces apoptosis specifically in cancer cells. Numerous anticancer agents that act by inhibiting the kinase activity have conventionally been developed; however, no anticancer agents that function by enhancing the kinase activity are known. Agents that activate Plk1 are considered to function as an anticancer agent according to a new mechanism of action.

In addition, it was confirmed that, in the abolishment of the suppressive binding of the kinase domain of Plk1 by PBD, a substance that causes the intramolecular structural conversion in Plk1 can be detected by not only the FRET assay but also the kinase assay. Further, the Plk1 activation can also be detected by phosphorylation. Accordingly, these systems can be used to screen compounds and peptides, and create new medicine in which the Plk1 activation is used as an indicator.

## Claims

1. A method for screening a medicine that targets polo-like kinase 1 (Plk1), in which Plk1 activation by addition of a candidate compound is used as an indicator.

2. The screening method according to claim 1, in which the Plk1 activation is due to inhibition of an association between a Plk1 kinase domain and a Polo-Box domain (PBD).

3. The screening method according to claim 1 or 2, in which the Plk1 activation is measured by A FRET assay, a kinase assay, or an antibody that detects phosphorylation.

4. The screening method according to claim 1, in which the medicine is for treating cancer.

5. A method for suppressing cancer cell growth, in which the growth is suppressed by activating Plk1.

6. A pharmaceutical composition for treating cancer, in which a compound that activates Plk1 is used as an active ingredient.

7. The pharmaceutical composition for treating cancer according to claim 6, in which a partner of numb (PON) peptide or a nucleic acid that encodes a PON peptide is used as an active ingredient.
